# EUROPEAN PATENT SPECIFICATION

(11) **EP 2 963 124 B1**
(45) Date of publication and mention of the grant of the patent: **20.06.2018**
(21) Application number: 14175297.2
(22) Date of filing: 01.07.2014
(51) Int. Cl.: C12Q 1/68, G01N 33/574

(54) **Biomarker combinations for use in pancreatic cancer screening**
Biomarkerkombinationen zur Verwendung beim Bauchspeicheldrüsenkrebs-Screening
Combinaisons de biomarqueurs utilisées dans le dépistage du cancer du pancréas

(43) Date of publication of application: 06.01.2016
(73) Proprietor: Randox Laboratories Ltd., Crumlin, County Antrim BT29 4QY (GB); University Of Lubeck, 23538 Lubeck (DE); Christian Albrechts University of Kiel, 24118 Kiel (DE); University Hospital Jena, 07743 Jena (DE); German Cancer Research Center (DKFZ), 69120 Heidelberg (DE)
(72) Inventor: Haberman, Jens, 23538 Lübeck (DE); Buenger, Stefanie, 23538 Lübeck (DE); von Eggeling, Ferdinand, 07743 Jena (DE); Brenner, Hermann, 69120 Heidelberg (DE); Freitag-Wolf, Sandra, 24105 Kiel (DE); McConnell, Ivan, Crumlin Antrim BT29 4QY (GB); Fitzgerald, Peter, Crumlin Antrim BT29 4QY (GB)

(56) References cited:
- STEFANIE BÜNGER ET AL: "A Novel Multiplex-Protein Array for Serum Diagnostics of Colorectal Cancer: Impact of Pre-analytical Storage Conditions", BIOPRESERVATION AND BIOBANKING, vol. 11, no. 6, 1 December 2013 (2013-12-01), pages 379-386, XP055140096, ISSN: 1947-5535, DOI: 10.1089/bio.2013.0050
- STEFANIE BÜNGER ET AL: "A novel multiplex-protein array for serum diagnostics of colon cancer: a case-control s", BMC CANCER, BIOMED CENTRAL, LONDON, GB, vol. 12, no. 1, 7 September 2012 (2012-09-07), page 393, XP021120505, ISSN: 1471-2407, DOI: 10.1186/1471-2407-12-393
- PATRICK CHAMES ET AL: "Therapeutic Antibodies for the Treatment of Pancreatic Cancer", THE SCIENTIFIC WORLD JOURNAL, vol. 10, 1 January 2010 (2010-01-01), pages 1107-1120, XP055140239, DOI: 10.1100/tsw.2010.103
- MING-BING XIAO ET AL: "High expression of S100A11 in pancreatic adenocarcinoma is an unfavorable prognostic marker", MEDICAL ONCOLOGY, SPRINGER-VERLAG, NEW YORK, vol. 29, no. 3, 13 September 2011 (2011-09-13), pages 1886-1891, XP035102306, ISSN: 1559-131X, DOI: 10.1007/S12032-011-0058-Y
- DANIELA CECCONI ET AL: "Proteomic analysis of pancreatic endocrine tumor cell lines treated with the histone deacetylase inhibitor trichostatin A", PROTEOMICS, vol. 7, no. 10, 1 May 2007 (2007-05-01), pages 1644-1653, XP055140149, ISSN: 1615-9853, DOI: 10.1002/pmic.200600811

## Description

### Introduction

Pancreatic cancer is one of the most lethal malignancies worldwide and thus demonstrates an urgent demand for improved screening tools for early detection. Detection of pancreatic cancer at early stages is crucial because successful surgery at early tumour stages is the only curative therapy today. Only 10 - 30% of pancreatic tumour patients are operated on with curative intent and a R0 resection can only be achieved for around half of these patients. The expected five-year-survival-rate of R0 resected patients with additional adjuvant chemotherapy is about 4 - 26% [2, 3]. In contrast, for the remaining patients who present with unresectable UICC stage III and IV carcinomas, no curative therapy exists. These patients have median survival times of 8 - 12 month (UICC III) and 5 - 8 month (UICC IV), respectively. Thereby, the incidence of this disease is almost identical to its mortality rate.

The persistent delay in diagnosis and the associated high mortality are attributable to the lack of symptoms at early tumour stages combined with a high biological aggressiveness of the tumour and limited treatment options. Therefore, improved screening for earlier diagnosis is essential in order to increase the rate of curatively resectable carcinomas and thereby ameliorate patients' prognosis. In present clinical practice, screening for pancreatic cancer is based on state-of-the-art imaging or even invasive diagnostics. Diagnostic modalities comprise endoscopic ultrasound (EUS), computed tomography (CT), endoscopic retrograde cholangiopancreatography (ERCP), magnetic resonance cholangiopancreatography (MRCP), magnetic resonance imaging (MRI), or even explorative laparoscopy/laparotomy. Any additional screening method improving early detection would have a highly beneficial impact on patients' prognosis.

A relatively non-invasive, cost-efficient possibility could be provided by the measurement of disease specific markers in peripheral blood. Identifying biomarkers derived from human serum is particularly appealing because blood can be obtained with high patient compliance and acceptability. Such a test can be envisioned if changes in the composition of serum proteins could indicate specific diseases and/or disease stages. Quantitative and qualitative serum protein profiling therefore have become a perceived goal of clinical proteomics in order to identify reliable diagnostic and prognostic biomarkers in clinical oncology.

A wide range of serum markers have been reported since the 1980s but only a few have shown promising results in recent studies. CA 19-9 is the most widely investigated and evaluated marker for pancreatic cancer detection. Serum CA 19-9 levels show a sensitivity of 79 - 81% at a specificity of 82 - 90% for the diagnosis of pancreatic cancer in symptomatic patients [6]. Further pancreatic cancer markers described in the prior art are CEA [12] and S100A11 [13]. most of the markers are yet to be validated in well defined, large screening studies. Hereby, the measurement of a disease specific panel of markers could outperform the measurement of individual markers regarding sensitivity and specificity. In addition, a marker panel could provide a more comprehensive reflection of molecular networks and pathophysiological conditions of diseases. Biochip array technology allows multiplex determination of multiple biomarkers from a single sample [4, 5]. This is also relevant when volumes of clinical samples are limited. Implementations of this technology in clinical settings have been reported for different biochip arrays including cytokines, cerebral and cardiac arrays, adhesion molecules and also drugs of abuse detection.

Considering recent single biomarker studies, serum level assessment of MIC-1, PAM4, OPN, HSP27, TPS, TSGF, CAM17.1, PF4 and CEACAM1 were reported as promising markers [7]. In addition, marker panels consisting of M-CSF and CA 19-9, CEA and CA 19-9, SAA, Haptoglobin and CA 19-9, TSGF, CA 242 and CA 19-9, and HSP27 and CA 19-9 seemed to be favourable for pancreatic carcinoma detection with high diagnostic potential. Despite research into these various single markers or marker combinations, none have been implemented for clinical routine use. Therefore, there is the need for new marker combinations to aid in the diagnosis and prognosis of pancreatic cancer.

### References

**1.** Benson AB, 3rd: JAMA 2007, 297(3):311-313.
**2.** Nieto J, Grossbard ML, Kozuch P: Oncologist 2008, 13(5):562-576.
**3.** Katz MH, Hwang R, Fleming JB, Evans DB: CA Cancer J Clin 2008, 58(2):111-125.
**4.** Molloy RM, Mc Connell RI, Lamont JV, FitzGerald SP: Clin Chem Lab Med 2005, 43(12): 1303-1313.
**5.** Fitzgerald SP, McConnell RI, Huxley A:. J Proteome Res 2008, 7(1):450-455.
**6.** Ballehaninna, U.K, Chamberlain, R.S. Indian journal of surgical oncology, 2011, 2(2):88-100.
**7.** Bünger S, Laubert T, Roblick UJ, Habermann JK: J Cancer Res Clin Oncol 2011, 137(3):375-389.
**8.** Bünger S, Haug U, Kelly M, Posorski N, Klempt-Giessing K, Cartwright A, Fitzgerald SP, Toner V, McAleer D, Gemoll T et al. BMC cancer 2012, 12:393.
**9.** Austin, P.C and Tu, J.V. The American Statistician, 2004, 58:131-137.
**10**.Streyerberg, E.W. Journal of clinical epidemiology, 2001, 54:774-781.
**11.** Bünger, S. et al. Biopreservation and Biobanking, 2013, 11(6):379-386
**12.** Chames et al. The Scientific World Journal 2010, 10:1107-1120
**13.** Xiao, Ming-Bing et al. Medical Oncology, 2011 29(3):1886-1891

### Summary of the Invention

In a first aspect, the current invention provides a novel biomarker combination for early diagnosis of pancreatic adenoma and carcinoma. The combination of M-CSF, S100A11, C3adesArg and CD26 displayed a 70% sensitivity at 90% specificity for the detection of pancreatic carcinomas. At the same specificity, even early carcinomas were detected with 69% sensitivity.

A second aspect of the current invention is a method for detecting pancreatic adenoma and/or carcinoma in an *in vitro* sample taken from a patient, wherein the sample is contacted with a solid- state device onto which has been immobilized probes specific to each of the biomarkers.

Another aspect described herein is a solid-state device comprising a substrate having an activated surface onto which is immobilized probes to M-CSF, S100A11, C3aDesArg and CD26 in discrete areas of said activated surface. This solid-state device not only has potential in diagnosis, but also in monitoring the progression of pancreatic cancer.

Another aspect described herein is a method of determining the efficacy of a drug treatment for pancreatic cancer comprising determining the levels of M-CSF, S100A11, C3aDesArg and CD26 in a sample from a patient treated with the drug, and comparing levels with those from a healthy control or with levels from the same patient before treatment with the drug, wherein dependent on the biomarker, either an increase or decrease in level indicates the effectiveness of the treatment.

### Description of the Drawings

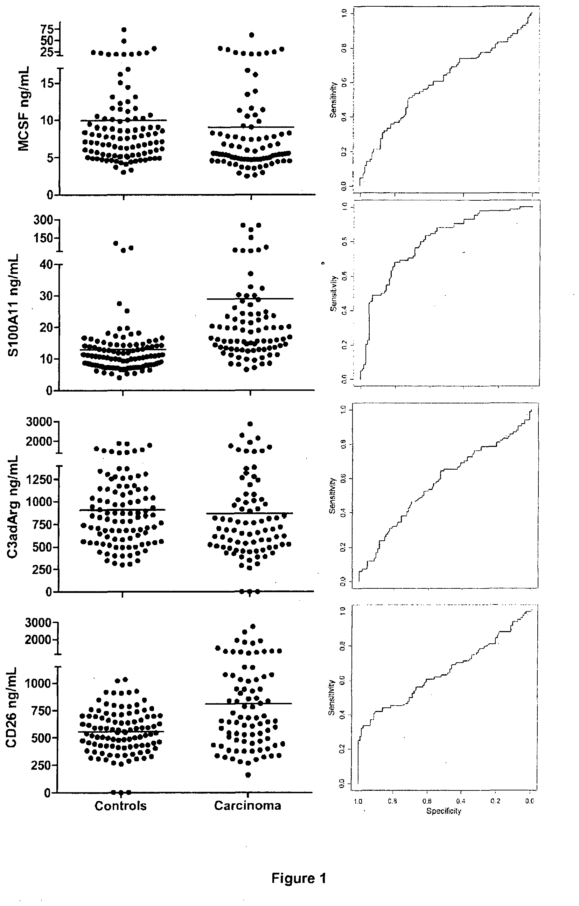

**Figure 1** - **Diagnostic performance of single markers for early detection of pancreatic cancer.**
   Single sample dot plots with mean serum levels and ROC curves regarding the discrimination between healthy controls and pancreatic carcinoma for M-CSF, S100A11, C3adesArg and CD26 in single markers performance.

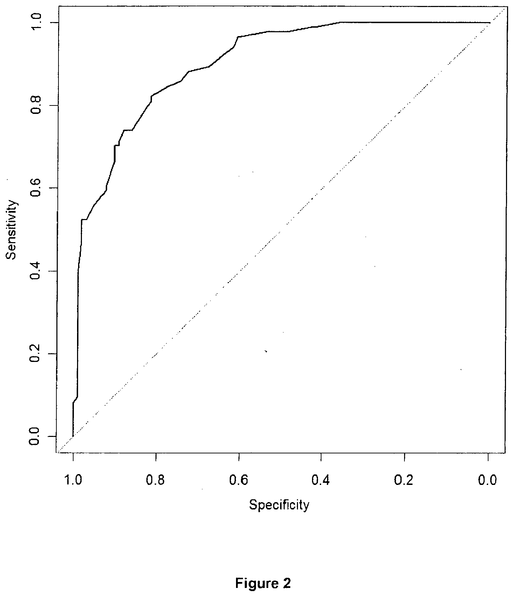

**Figure 2** - **Diagnostic performance of marker combination for detection of pancreatic** cancer. Receiver operating characteristics curve regarding the discrimination between pancreatic cancer and controls for a predictive model based on biomarkers M-CSF, S100A11, C3adArg, CD26 in combination. AUC = 0.9015 (95%Cl: 0.8595 - 0.9435).

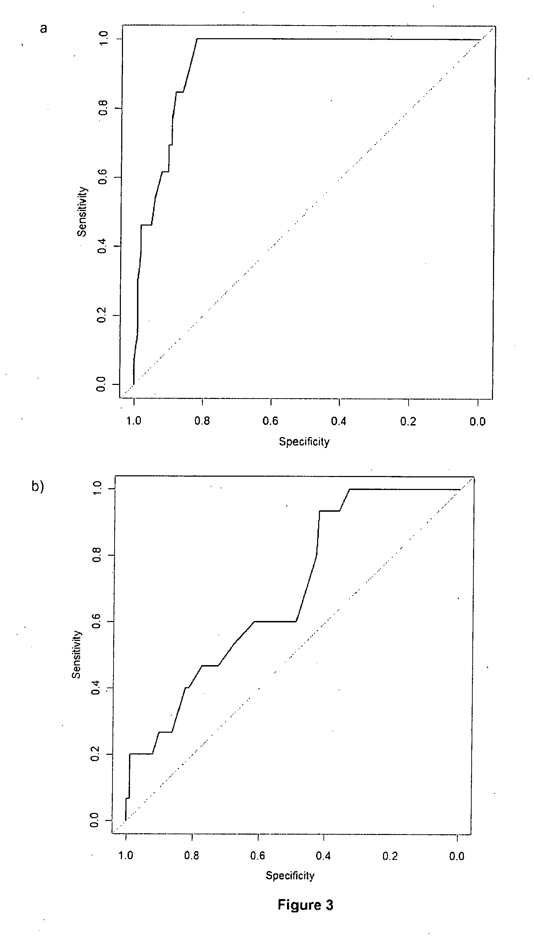

**Figure 3** - **Diagnostic performance of marker combination for pancreatic adenomas and early carcinomas.** Receiver operating characteristics curve regarding the discrimination between **a)** early carcinomas and controls (AUC = 0.937, 95%Cl: 0.892 - 0.983) and **b)** pancreatic adenoma and controls (AUC = 0.687, 95%Cl: 0.554 - 0.820) for a predictive model based on biomarkers M-CSF, S100A11, C3adArg, CD26 in combination.

### Detailed Description

The present invention describes a biomarker-based method to aid in the early diagnosis of pancreatic cancer. Specifically the measurement of relative levels or concentrations of biomarkers *in vitro* from a sample taken from a patient are measured. In the context of the current invention, the utility for diagnosing pancreatic cancer has been used as way of an example. However it is further envisaged that the invention may also be used for monitoring the progression or recurrence of pancreatic cancer and also the effectiveness of any treatment strategy which has been implemented. The current invention can also be used to determine persons at risk of developing pancreatic cancer, for example by aiding in the diagnosis of pancreatic adenoma, a pre-malignant condition that may develop into pancreatic cancer.

The term "patient" refers to any mammal which is the recipient of the diagnosis, preferably a human. The patient may be a person presenting for routine screening for disease or they may present with symptoms suggestive of cancer. The patient may also be an individual deemed at high risk for pancreatic cancer, due to family history for example. Alternatively, the patient could be an individual who has received treatment for pancreatic cancer and the screen is to monitor progress or detect possible recurrence.

The term "biomarker", in the context of the current invention, refers to a molecule present in a biological sample of a patient, the levels of which may be indicative of pancreatic cancer or adenoma. Such molecules may include peptides/proteins or nucleic acids and derivatives thereof.

The biomarker combination of the current invention for the detecting of pancreatic cancer comprises S100A11, M-CSF, C3adesArg and CD26. However it is within the scope of the invention to determine levels of additional biomarkers which could contribute to a diagnosis of pancreatic cancer, for example, but not limited to, CA 19-9, CEA, IL-8, VEGF and CRP. In another aspect combinations of two or three biomarkers can be used in a method to determine if a patient has or is at risk of developing pancreatic cancer are described.

These biomarkers are preferably selected from the list consisting of M-CSF, S100A11, C3adesArg, CD26, CA 19-9, CEA, IL-8, VEGF and CRP. Most preferably, one of the biomarkers in a two or three biomarker combination is S100A11. Examples of preferred two biomarker combinations include S100A11 and VEGF, S100A11 and CRP, S100A11 and CEA, S100A11 and CD26, S100A11 and M-CSF, S100A11 and C3adesArg, S100A11 and IL-8.

The term "M-CSF" as used herein refers to Macrophage colony-stimulating factor, also known as colony-stimulating factor 1 (UniProt number P09603).

The term "S100A11" as used herein refers to S100 calcium binding protein A11 (UniProt number P31949), also known as calgizzarin.

The term "C3adesArg" as used herein refers to the cleavage product of complement C3 (UniProt number P01024) which is also commonly referred to as acylation-stimulating protein (ASP).

The term "CD26" as used herein refers to cluster of differentiation 26, also known as Dipeptidyl peptidase 4 (DPP-4) (UniProt number P27487).

The term "CEA" as used herein refers to carcinoembryonic antigen (UniProt number P06731), also known as carcinoembryonic antigen-related cell adhesion molecule 5.

The term "VEGF" as used herein refers to vascular endothelial growth factor (UniProt number P15692).

The term "CRP" as used herein refers to C-reactive protein (UniProt number P02741).

The term "IL-8" as used herein refers to interleukin 8 (UniProt number P10145).

In the context of the present invention, a deviation from a control value for each of the biomarkers may be an indication that the patient suffers from pancreatic adenoma or pancreatic carcinoma. Dependent on the individual biomarker this deviation may be an increase or a decrease from a control value. For example, in the patient cohort of the current invention, levels of M-CSF and C3adesArg were lower in adenoma and carcinoma patients when compared to those in healthy controls. Levels of S100A11 and CD26 were higher in adenoma and carcinoma patients than in healthy controls (table 2).

The current invention provides an early stage biomarker combination, which allows the detection of neoplastic disease at an early and still benign stage and/or early tumour stages. Early detection and removal of a pancreatic adenoma or carcinoma is critical and can dramatically increase the patients' chances of survival. Additionally the biomarker combination of the current invention allows the monitoring of pancreatic adenoma or carcinomas development within an individual through serial testing of serum from said individual over an extended period. For example, routine determination of the levels of the four biomarkers of the preferred combination could detect the changes from healthy control values, which are indicative of the development of pancreatic adenoma and/or early stage pancreatic carcinoma. A further change in levels could then be indicative of the progression of the disease to a later stage.

A further aspect is a method of determining the efficacy of a treatment for pancreatic cancer comprising determining the levels of two or more biomarkers selected from the list consisting of S100A11, M-CSF, C3aDesArg, CD26, CA 19-9, CEA, IL-8, VEGF and CRP in a sample from a treated patient, and comparing levels with those from a healthy control or with levels from the same patient before treatment, wherein dependent on the biomarker, either an increase or decrease in level indicates the effectiveness of the treatment. The treatment can be for example, a drug treatment, a radiotherapy based treatment or a surgical intervention. In a preferred method for determining the efficacy of a treatment for pancreatic cancer, one of the two or more markers is S100A11. In a further preferred method for determining the efficacy of a treatment for pancreatic cancer the two or more biomarkers consist of S100A11, M-CSF, C3adesArg and CD26. Wherein the treatment is a drug treatment, the method of determining the efficacy of the drug treatment for pancreatic cancer would comprise determining the levels of biomarkers, for example S100A11, M-CSF, C3adesArg and CD26, in a sample from a patient treated with the drug, and comparing biomarker levels with those from a healthy control or with levels from the same patient before treatment with the drug, wherein, dependent on the biomarker, either an increase or decrease in level indicates the effectiveness of the drug treatment.

In the context of the present invention, a "control value" is understood to be the level of a particular biomarker, such as S100A11, M-CSF, C3adesArg or CD26 typically found in healthy individuals. The control level of a biomarker may be determined by analysis of a sample isolated from a healthy individual or may be the level of the biomarker understood by the skilled person to be typical for a healthy individual. The control value may be a range of values considered by the skilled person to be a normal level for the biomarker in a healthy individual. The skilled person will appreciate that control values for a biomarker may be calculated by the user analysing the level of the biomarker in a sample from a healthy individual or by reference to typical values provided by the manufacturer of the assay used to determine the level of biomarker in the sample.

The "sample" can be any ex vivo biological sample from which the levels of biomarkers can be determined. Preferably, the sample isolated from the patient is a serum or plasma sample. However, the sample could be selected from, for example, whole blood, urine, saliva or sputum. The determination of the level of biomarkers may be carried out on one or more samples taken from the patient. The sample may be obtained from the patient by methods routinely used in the art.

The determination of the level of biomarkers in the sample may be determined by immunological methods such as an ELISA-based assay. The methods of the current invention preferably comprise the following steps; the biomarkers binding to a probe(s), adding a detector probe(s) and detecting and measuring the biomarker/probe complex signal(s), placing these values into a machine algorithm and analysing the output value, said value indicating whether the patient has or is at risk of having pancreatic cancer. Preferably, the methods of the present invention use a solid-state device for determining the level of biomarkers in the sample isolated from the patient.

The solid-state device comprises a substrate having a probe or multiple different probes immobilised upon it that bind specifically to a biomarker. The interactions between a biomarker and its respective probe can be monitored and quantified using various techniques that are well-known in the art. The term "probe" refers to a molecule that is capable of specifically binding to a target molecule such that the target molecule can be detected as a consequence of said specific binding. Probes that can be used in the present invention include, for example, antibodies, aptamers, phages and oligonucleotides. In a preferred embodiment of the current invention the probe is an antibody.

The "level" of a biomarker refers to the amount, expression level or concentration of the biomarker within the sample. This level can be a relative level in comparison to another biomarker or a previous sample.

The term "antibody" refers to an immunoglobulin which specifically recognises an epitope on a target as determined by the binding characteristics of the immunoglobulin variable domains of the heavy and light chains (VHS and VLS), more specifically the complementarity-determining regions (CDRs). Many potential antibody forms are known in the art, which may include, but are not limited to, a plurality of intact monoclonal antibodies or polyclonal mixtures comprising intact monoclonal antibodies, antibody fragments (for example Fab, Fab', and Fv fragments, linear antibodies single chain antibodies and multispecific antibodies comprising antibody fragments), single-chain variable fragments (scFvs), multi-specific antibodies, chimeric antibodies, humanised antibodies and fusion proteins comprising the domains necessary for the recognition of a given epitope on a target. Preferably, references to antibodies in the context of the present invention refer to polyclonal or monoclonal antibodies. Antibodies may also be conjugated to various reporter moieties for a diagnostic effect, including but not limited to radionuclides, fluorophores, dyes or enzymes including, for example, horse-radish peroxidase and alkaline phosphatase.

Such antibodies may be immobilised at discrete areas of an activated surface of the substrate. The solid-state device may perform multi-analyte assays such that the level of a biomarker in a sample isolated from the patient may be determined simultaneously with the level of a further biomarker of interest in the sample. In this aspect, the solid-state device has a multiplicity of discrete reaction sites each bearing a desired antibody covalently bound to the substrate, and in which the surface of the substrate between the reaction sites is inert with respect to the target biomarker. The solid-state, multi-analyte device may therefore exhibit little or no non-specific binding. The combination of biomarkers may also be referred to as a panel of biomarkers.

The substrate can be any surface able to support one or more probes, but is preferably a biochip. A biochip is a planar substrate that may be, for example, mineral or polymer based, but is preferably ceramic. When identifying the various biomarkers/proteins of the invention it will be apparent to the skilled person that as well as identifying the full-length protein, the identification of a fragment or several fragments of a protein is possible, provided this allows accurate identification of the protein. Similarly, although a preferred probe of the invention is a polyclonal or monoclonal antibody, other probes such as aptamers, molecular imprinted polymers, phages, short chain antibody fragments and other antibody-based probes may be used.

A solid-state device that may be used in the invention may be prepared by activating the surface of a suitable substrate and applying an array of antibodies on to the discrete sites on the surface. If desired, the other active areas may be blocked. The ligands may be bound to the substrate via a linker. In particular, it is preferred that the activated surface is reacted successively with an organosilane, a bi-functional linker and the antibody. The solid-state device used in the methods of the present invention may be manufactured according to the method disclosed in, for example, GB-A-2324866 the contents of which are incorporated herein in its entirety. The solid-state device can be any substrate to which probes of the current invention can be attached for example a microtitre plate or beads. Preferably, the solid-state device used in the methods of the present invention is a biochip. The biochip may be a biochip which is incorporated into the Biochip Array Technology System (BAT) available from Randox Laboratories Limited (Crumlin, UK).

Preferably, a solid-state device may be used to determine the levels of two or more biomarkers selected from the list comprising S100A11, M-CSF, C3adesArg, CD26, CA 19-9, CEA, IL-8, VEGF and CRP in the sample isolated from the patient. In a further aspect one of the two or more biomarkers is S100A11. In an embodiment the combination of biomarkers comprises S100A11, M-CSF, C3adesArg and CD26. In another aspect the solid-state device comprises a substrate having an activated surface on to which is applied antibodies specific to each of the two or more biomarkers to discrete areas of the activated surface. Therefore, the solid-state device may perform multi-analyte assays such that the levels of biomarkers, for example S100A11, M-CSF, C3adesArg and CD26, in a sample may be determined simultaneously. In this aspect, the solid-state device has a multiplicity of discrete reaction sites each bearing a desired antibody covalently bound to the substrate, and in which the surface of the substrate between the reaction sites is inert with respect to the target biomarkers. Each probe, whether individually or in multiplex, is specific to one target analyte. For example a probe to S100A11 will only show specific binding to this analyte and will have no significant cross-reactivity with M-CSF, C3adesArg, CD26, CA 19-9, CEA, IL-8, VEGF or CRP, or indeed any other potentially interfering substance which could compromise the assay. The solid-state device not only has potential in diagnosis but also in monitoring the progression and recurrence of pancreatic cancer as well as determining the success of any treatments.

In a preferred embodiment of the current invention each of the biomarker concentration values is inputted into a statistical methodology to produce an output value that correlates with the chances that the patient has or is at risk of developing pancreatic cancer. Preferably, the statistical methodology used is logistic regression, decision trees, support vector machines, neural networks, random forest or another machine-learning algorithm. The performance of the results of the applied statistical methods used in accordance with the present invention can be best described by their receiver operating characteristics (ROC). The ROC curve addresses both the sensitivity (the number of true positives) and the specificity (the number of true negatives) of the test. Therefore, sensitivity and specificity values for a given combination of biomarkers are an indication of the performance of the test. For example, if a biomarker combination has a sensitivity and specificity value of 80%, out of 100 patients, 80 will be correctly identified from the determination of the presence of the particular combination of biomarkers as positive for the disease, while out of 100 patients who do not have the disease 80 will accurately test negative for the disease.

A suitable statistical classification model, such as logistic regression, can be derived for a combination of biomarkers. Moreover, the logistic regression equation can be extended to include other (clinical) variables such as age and gender of the patient as well. In the same manner as described before, the ROC curve can be used to access the performance of the discrimination between patients and controls by the logistic regression model. Therefore, the logistic regression equation can be used apart or combined with other clinical characteristics to aid clinical decision making. Although a logistic regression equation is a common statistical procedure used in such cases and is preferred in the context of the current invention, other mathematical/statistical, decision trees or machine learning procedures can also be used.

One convenient goal to quantify the diagnostic accuracy of a laboratory test is to express its performance by a single number. The most common global measure is the area under the curve (AUC) of the ROC plot. The area under the ROC curve is a measure of the probability that the perceived measurement will allow correct identification of a condition. Values typically range between 1.0 (perfect separation of the test values of the two groups) and 0.5 (no apparent distributional difference between the two groups of test values), any result below 0.5 is considered to be worse than a random guess. The area does not depend only on a particular portion of the plot such as the point closest to the diagonal or the sensitivity at 90% specificity, but on the entire plot. This is a quantitative, descriptive expression of how close the ROC plot is to the perfect one (area = 1.0). In the context of the present invention, the two different conditions can be whether a patient has or does not have cancer.

### Materials and Methods

### Study group

This study comprised 201 serum samples that were part of altogether 3,500 serum samples collected at the University Medical Center Schleswig-Holstein, Campus Lübeck, Germany, between 2007 and 2012. This serum collection is part of the Hospital-integrated, centralized biobank *Interdisciplinary Center for Biobanking* - *Lübeck* (ICB-L), the *Surgical Center for Translational Oncology* - *Lübeck* (SCTO-L), University of Lübeck, and the German Cancer Aid (DKH e.V.) funded network *North German Tumorbank of Colorectal Cancer* (ColoNet, #108446). Serum samples were collected adhering to the guidelines of the local ethical review board (*Medical University of Lübeck, #07-124*) and according to strict standard operation procedures (see below). Serum samples were obtained prior to oncologic resection. For control patients, blood samples were obtained before full colonoscopy, which confirmed that no signs of premalignant or malignant intestinal lesions were present in this cohort. For cancer patients, blood samples were obtained before neoadjuvant chemo- or radiotherapy and/or surgery. The study group comprised 84 patients with pancreatic cancer (46 men and 38 women, mean age 64 years). Additionally 15 pancreatic adenoma patients (7 men and 8 women, mean age 59 years) und 102 healthy control patients (40 men and 62 woman, mean age 62 years) were included in this study. Detailed clinical data of the study group are summarized in **Table 1 and 4.**

### Sampling

All venous blood samples were obtained using serum gel-monovettes (*#01.1602, Sarstedt AG & Co, Nümbrecht, Germany*) and centrifuged at 1,550 x g for 10 min at 4 °C to separate the serum. Aliquots of serum samples were stored at -70°C within 30 min after venous puncture. Samples were thawed on ice before multiplex assessment on the biochips.

### Determination of biomarker serum levels

An established multiplex biochip platform design for the simultaneous quantitative detection of the serum markers IL-8, CEA, VEGF, M-CSF, S100A11, C3adesArg, CD26 and CRP was designed and manufactured as previously described [8,9,11]. Thereby, one biochip (CRCSI) comprised C3desArg, CD26 and CRP, and the other chip (CRCSII) incorporated IL-8, CEA, VEGF, M-CSF, S100A11 and NNMT. The biochips were based on simultaneous chemiluminescent sandwich immunoassays using the *Evidence Investigator* analyser (*Randox Laboratories Ltd., Crumlin, UK*). This system allows handling of up to 54 samples (6 x 9 wells) in one biochip carrier. Samples were thawed on ice before multiplex assessment. The biochip undergoes 100% quality control assessment after manufacture avoiding the need for technical replicates for patient samples. The inclusion of calibrators and controls provides confidence in the inter-assay results reported. The analyte concentration present in the sample was calculated automatically using generated calibration curves (*Evidence Investigator Software version 1.4*). The analyser routinely assesses the quality of assay performance as described by FitzGerald et al. [5]. The detailed protocol for chip incubation and processing as well as assay ranges and accuracy were described previously [9].

### Statistical analysis

Using SPSS Statistics version 19 (*IBM Corporation, Somer, NY*) and SAS version 9.1 (*SAS Institute Inc., Cary, North Carolina, USA*), serum levels of the individual markers in pancreatic cancer cases, pancreatic adenoma cases and healthy controls were described with respect to median levels, interquartile ranges and overall ranges. Nonparametric tests to compare median serum levels between different patient groups were applied. The correlation between the single markers was assessed using Spearman's correlation coefficient.

Further statistical analyses regarding clinical performance of the biomarkers was conducted using R version 2.12.2, packages bootStepAIC, pROC and MASS as follows: The relationship between disease and all eight diagnostic markers was analysed separately by logistic regression models and illustrated by receiver operating characteristic (ROC) curves. Since it turned out that the continuous variables yielded a non-linearity on the logistic scale, all markers were classified into quartiles for further modelling. A predictive model was built by backward selection according to the AIC criteria. Additionally, we excluded IL-8 from the predictive model, because there was no monotonicity in quartiles in respect to disease status detectable. The stability of the marker selection was validated by bootstrap methods for developing predictive models of Austin and Tu [9].

To achieve robust estimators of the odds ratio (OR) with appropriate confidence intervals (CI) of the final model the 50th, 2.5th, and 97.5th percentile of estimated regression parameters of 10.000 logistic regression models from bootstrap samples of the original data set were used. P-values were calculated from the inversion of two-sided confidence regions, which are associated with testing the two-sided null hypothesis that the OR equals zero. All bootstrap results from the final model were compared to results from asymptotic theory.

The internal validation of the final model was conducted by an enhanced bootstrap method [10]. The optimism in the apparent performance was evaluated by averaging 1000 differences of two area under the receiver operating characteristic curve (AUC) values: AUC values calculated when the same bootstrap sampling was applied (apparent performance) and when the original data set was applied to the model estimated from the bootstrap data (test performance).

### Examples

Biochips and targets were simultaneously evaluated in a highly standardized serum collection of cancer, pre-malignancies and control samples stored at -70 °C within 30 min after phlebotomy following strict standard operation procedures.

### Example 1 - Evaluation of single markers

The distribution of serum levels of all eight markers is described in **Table 2.** Serum levels of CEA, VEGF, M-CSF, S100A11, CD26 and CRP showed significant differences between pancreatic cancer cases and controls (P < 0.05). Corresponding single dot plots and ROCs of the four superior single markers are shown in **Figure 1****.** AUCs with diagnostic performances for all eight markers are listed in **Table 3a:** The largest AUC was observed for S100A11 (0.799). S100A11 was also the marker with the highest sensitivity of 49% at levels of specificity that are typically required in a screening setting (≥ 90%).

### Example 2 - Development of a predictive regression model

In order to optimize the clinical performance of pancreatic cancer detection, we tested if a marker combination would be superior over single marker use. After backward selection, the final model comprised four biomarkers M-CSF, S100A11, C3desArg and CD26 (**Table 3b**). Asymptotic results (not shown) were almost very similar but in some cases less conservative than those from bootstrap methods. Verifying variable selection on bootstrap samples demonstrates that it clearly distinguishes between important and unimportant predictive markers in the final model. Thus, the bootstrap model confirms, that our model is based on those four serum markers with the highest impact. The corresponding AUC of 0.9015 (95%Cl: 0.8595 - 0.9435) obtained e.g. 70% sensitivity at 90% specificity. Thereby, the highest impact was detected for S100A11 serum levels > 18.81 ng/mL (OR 286.7) and for CD26 serum levels > 795 ng/mL (OR 21.6, **Table 3b**).

For further validation, the optimism of our model was performed as described above. The average of the AUC from the same sample from the model was built was 0.911 (95%Cl: 0.86 - 0.912), whereas for a new bootstrap sample the average of the AUC was 0.89 (95%Cl: 0.852 - 0.901). The mean of the optimism is 0.02 (95%Cl: -0.025 - 0.059). With an included model selection negligible changes are drawn. This confirms the stability of our regression model for pancreatic cancer detection.

Furthermore, we developed a corresponding formula for clinical application of the predictive model. A linear combination (LC) was calculated including regression factors for all marker quartiles being part in the predictive model: *LC* = *-1.4* - *1.2 x I__{M-CSFq1}* - *2.7 x I__{M-csFq2}* - *1.8 x I__{M-CSFq4}* +*1.5 x I__{S100A11q1}* +*2.2 x I_{-S100A11q2}* +*5.3 x I__{S100A11q3} -1.7 x I__{C3adesArgq3} +2.9 x I__{CD26q3}.* For each marker individual serum values of the patient need to be checked and converted into 0 if the serum level is not in the corresponding quartile, otherwise 1 should be inserted if serum level ranges are in the corresponding quartile. Consequently, for each *I__{MarkerXqx}* 0 or 1 is inserted into the formula. Subsequently, the *LC* value is then further processed in the formula *P_{PC}* = *1*/ *(1* + *exp (-LC))* resulting in the probability of being affected by pancreatic cancer. Exemplary, we observed a patient (#912) with the following serum levels for the four markers included in the predictive model: M-CSF = 8.29 ng/mL (quartile 2),S100A11= 14.89 ng/mL (quartile 2), C3adesArg = 914 ng/mL (quartile 0) and CD26 = 786 ng/mL (quartile 0). The calculation of the LC consequently is -1.4 - (1.2 x 0) - (2.7 x 1) - (1.8 x 0) + (1.5 x 0) + (2.2 x 1) + (5.3 x 0) - (1.7 x 0) + (2.9 x 0) = -1.033 and the probability therefore could be computed with *P_{PC}* = 1/ (1+exp(1.033) = 0.27 and thereby 27% for having pancreatic cancer.

### Example 3 - Serum levels and test performance for early stage carcinomas

When comparing serum levels between all early stage (UICC stages I and II, n=13) and late stage pancreatic carcinomas (UICC stages III and IV, n=69), the median serum level for CEA was 0.44 ng/mL in early stage carcinomas and 1.06 ng/mL in late stage carcinomas (P = 0.028). The serum levels for all other markers showed no significant differences between early and late stage carcinomas (**Table 2**). Applying the previously described predictive model to 13 early stage carcinomas, the sensitivity was 69% at 90% specificity (AUC = 0.937, 95%Cl: 0.892 - 0.983, **Figure 3a**). Thus, reaching the same sensitivity as for the 69 late stage carcinomas (69% sensitivity at 90% specificity, AUC = 0, 892, 95%Cl: 0.845 - 0.938, **Figure 3a**).

### Example 4 - Serum levels and test performance for adenomas

The 15 pancreatic adenoma cases histologically consisted of five cyst-adenomas, four intraductal papillary mucinous neoplasms (IPMNs), four others (cystic, papillar, mucionous, endocrine type) and two adenomas of unknown histology (for detailed clinical data see **Table 4).** For IL-8 the median serum level for patients with adenomas was significantly lower than the levels observed in all healthy controls (9.32 pg/mL vs. 19.12 pg/mL, P = 0.002). Similar results were shown by serum levels of M-CSF that were lower in adenoma compared to healthy patients (5.71 ng/mL vs. 7.66 ng/mL, P = 0.009). In contrast, S100A11 showed a higher median level for adenoma than for healthy patients (15.65 ng/mL vs. 10.69 ng/mL, P < 0.0001). All other markers did not significantly differ between adenoma and healthy patients. The biochip results for all adenomas are included in **Table 2.** For adenoma samples, our multiplex panel of S100A11, M-CSF, C3adesArg and CD26 reached 27% sensitivity at 90% specificity (AUC = 0.687, 95%Cl: 0.554 - 0.820, **Figure 3b**).

### Example 5 - Correlation among biomarker levels and clinicopathological features

Sex and age were distributed equally between individuals of all groups (P = 0.107 and 0.250, respectively). In order to explore whether the serum levels of the four multiplex panel serum markers showed a correlation with age or sex, Spearman's correlation coefficients were calculated. M-CSF and CD26 showed a correlation to age (Spearman's correlation coefficient ρ = 0.261, P < 0.001 and ρ = -0.192, P = 0.009, respectively), while S100A11 was correlated with sex (ρ = -0.238, P = 0.001). There was no significant correlation between all other biomarkers and age or sex. There were also no correlations of biomarker levels to T-, N- or M-status or grading of the carcinoma (Spearman Correlation and Mann-Whitney Test).

Regarding M-CSF, S100A11, C3adesArg and CD26 no relevant correlation among each other could be detected applying Spearman's correlation coefficient (all ρ < 0.300, **Table 5**).

**Table 1 - Clinical Data of the study group**

| ***a)** Summary of clinical data of the study group consisting of pancreatic cancer patients, pancreatic adenoma patients and healthy control patients.* | | | | | |
|---|---|---|---|---|---|
| ***Parameter*** | ***Value*** | ***Pancreatic cancer patients (n*=*84)*** | ***Pancreatic adenoma patients (n*=*15)*** | ***Healthy control patients (n*=*102)**** | ***P value*** |
| ***Sex*** | *Female* | *38 (45 %)* | *8 (47 %)* | *62 (61 %)* | *0.107* |
| | *Male* | *46 (55 %)* | *7 (53 %)* | *40 (39 %)* | |
| ***Age (years)*** | *Range* | *64* | *59* | *62* | *0.250* |
| | *Mean* | *37* - *83* | *42 - 72* | *20* - *91* | |

| *b) Summary of clinical data of pancreatic cancer patients. n.a.: not available, because of carcinoma were inoperable.* | | | | | |
|---|---|---|---|---|---|
| ***Parameter*** | ***Value*** | ***Pancreatic cancer patients n (**%**)*** | ***Pancreatic adenoma patients n (%)*** | | |
| ***Tumor localization*** | *head* | *56 (66.7%)* | *2 (13.3%)* | | |
| | *corpus* | *13 (15.5%)* | *2 (13.3%)* | | |
| | *tail* | *4 (4.8%)* | *0 (0.0%)* | | |
| | *n.a.* | *11 (13.1%)* | *11 (73.3%)* | | |
| ***UICC stage*** | *1* | *4 (4.8%)* | | | |
| | *2* | *9 (10.7%)* | | | |
| | *3* | *42 (50%)* | | | |
| | *4* | *27 (32.1%)* | | | |
| | *n.a.* | *2 (2.4%)* | | | |
| ***T stage*** | *1* | *4 (4.8%)* | | | |
| | *2* | *12 (14.3%)* | | | |
| | *3* | *37 (44%)* | | | |
| | *4* | *17 (20.2%)* | | | |
| | *n.a.* | *14 (16.7%)* | | | |
| ***Lymphnode stage*** | *0* | *14 (16.7%)* | | | |
| | *1* | *44 (52.4%)* | | | |
| | *2* | *2 (2.2%)* | | | |
| | *n.a.* | *24 (18.6%)* | | | |
| ***Metastasis stage*** | *0* | *18 (21.4%)* | | | |
| | *1* | *14 (16.7%)* | | | |
| | *n.a.* | *52 (61.9%)* | | | |
| ***Tumor grading*** | *G1*/*2* | *25 (29.8%)* | | | |
| | *G3* | *26 (31%)* | | | |
| | *n.a.* | *33 (39.3%)* | | | |

| | | | | | |
|---|---|---|---|---|---|
| *All healthy patients received a full colonoscopy | | | | | |

**Table 2 - Serum level of eight serum biomarkers**

| *Median serum levels of eight biomarkers in healthy, adenoma and (early and late) pancreatic carcinoma patients. ns: not significant (p > 0.05),* * *Detection Limit,* ^{•} *comparison between healthy and adenoma patients* | | | | | |
|---|---|---|---|---|---|
| | ***Healthy patients*** | ***Cancer patients*** | ***Adenoma patients*** | ***UICC stage*** | |
| | | | | ***I* + *II*** | ***III* + *IV*** |
| | ***n*** = ***102*** | ***n*** = ***84*** | ***n*** = ***15*** | ***n*=*13*** | ***n*=*69*** |
| ***CEA (ng*/*mL)*** | | | | | |
| *Median* | *0.55* | *1.01* | *0.65* | *0.44* | *1.06* |
| *Interquartile range* | *0.36 - 1.13* | *053* - *1.96* | *0.29 - 1.63* | *0.375* - *1.2* | *0.58* - *2.06* |
| *Range* | *0.14 - 6.05* | *0.14* - *52.39* | *0.2 - 3.79* | *0.26* - *3* | *0.14 - 52.39* |
| *p-value* | *< **0.0001*** | | *ns^{•}* | ***0.028*** | |

| ***IL-8 (pg*/*mL)*** | | | | | |
|---|---|---|---|---|---|
| *Median* | *19.12* | *20.12* | *9.32* | *17.89* | *20.16* |
| *Interquartile range* | *15.73* - *25.41* | *12.24* - *38.45* | *8.01 - 14.02* | *12.49 - 12.49* | *11.93* - *34.81* |
| *Range* | *0 - 221.15* | *0 - 465.33* | *3.1 - 27.86* | *8.3 - 313.35* | *0* - *465.33* |
| *p-value* | *ns* | | ***0.002*** | *ns* | |

| ***VEGF (pg*/*mL)*** | | | | | |
|---|---|---|---|---|---|
| *Median* | *57.04* | *103.78* | *81.36* | *105.75* | *99.58* |
| *Interquartile range* | *33.99* - *98.45* | *50.07* - *154.82* | *54.5* - *129.64* | *45.99* - *129.62* | *52.48* - *165.86* |
| *Range* | *7.54* - *427.56* | *21.22 - 530** | *28.05* - *170.25* | *34.11* - *238.84* | *21.22* - *530** |
| *p-value* | *< **0.0001*** | | *ns^{•}* | *ns* | |

| ***M-CSF (ng*/*mL)*** | | | | | |
|---|---|---|---|---|---|
| *Median* | *7.66* | *5.58* | *5.71* | *7.49* | *5.56* |
| *Interquartile range* | *5.33 - 10.33* | *4.73 - 9.15* | *3.26* - *8.08* | *5.36 - 74.02* | *4.7* - *8.28* |
| *Range* | *2.99* - *72.86* | *2.47* - *60.69* | *2.64* - *10.28* | *4.43* - *32.09* | *2.47* - *60.90* |
| *p-value* | ***0.015*** | | ***0.009^{•}*** | *ns* | |

| ***S700A11 (ng*/*mL)*** | | | | | |
|---|---|---|---|---|---|
| *Median* | *10.69* | *16.72* | *15.65* | *15.34* | *16.61* |
| *Interquartile range* | *767 - 14.22* | *13.19* - *24.31* | *13.06* - *18.41* | *12.87* - *29.71* | *13.37 - 24.26* |
| *Range* | *3.99 - 105.25* | *6.52* - *250** | *10.65* - *34.66* | *9.8* - *250* | *6.52 - 250** |
| *p-value* | *< **0.0001*** | | ***< 0.0001^{•}*** | *ns* | |

| ***C3adesArg(ng*/*mL)*** | | | | | |
|---|---|---|---|---|---|
| *Median* | *845* | *692* | *640* | *528* | *690* |
| *Interquartile range* | *575.5* - *1146.5* | *498 - 1021* | *574 - 966* | *440* - *1152* | *504 - 1020* |
| *Range* | *300 - 3316* | *0* - *4392* | *0 - 3060* | *0 - 2860* | *0* - *4392* |
| *p-value* | *ns* | | *ns^{•}* | *ns* | |

| ***CD 26(ng*/*mL)*** | | | | | |
|---|---|---|---|---|---|
| *Median* | *550* | *667* | *618* | *840* | *646* |
| *Interquartile range* | *422.5 - 702.5* | *452.5* - *1051.5* | *428 - 704* | *542 - 1133* | *431* - *1023* |
| *Range* | *0 - 1034* | *160 - 2744* | *398 - 796* | *300 - 1928* | *160 - 2744* |
| *p-value* | ***0.001*** | | *ns^{•}* | *ns* | |

| ***CRP (ng*/*mL)*** | | | | | |
|---|---|---|---|---|---|
| *Median* | *1941* | *4654* | *2604* | *4720* | *4592* |
| *Interquartile range* | *1136.5* - *5030.5* | *1984.5* - *15909.5* | *1398 - 6398* | *2322* - *15846* | *1705 - 16754* |
| *Range* | *746* - *59800** | *950* - *59800** | *680 - 10590* | *1036* - *59800** | *950 - 59800** |
| *p-ralue* | *< **0.0001*** | | *ns^{•}* | *ns* | |

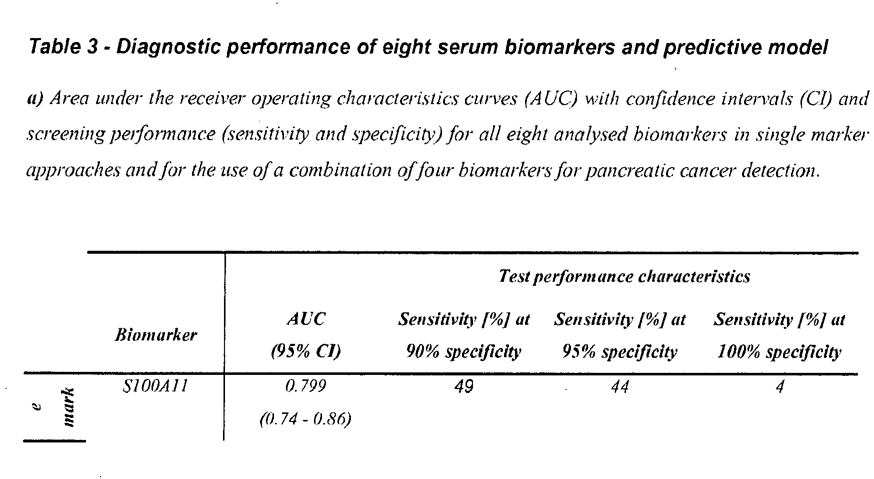

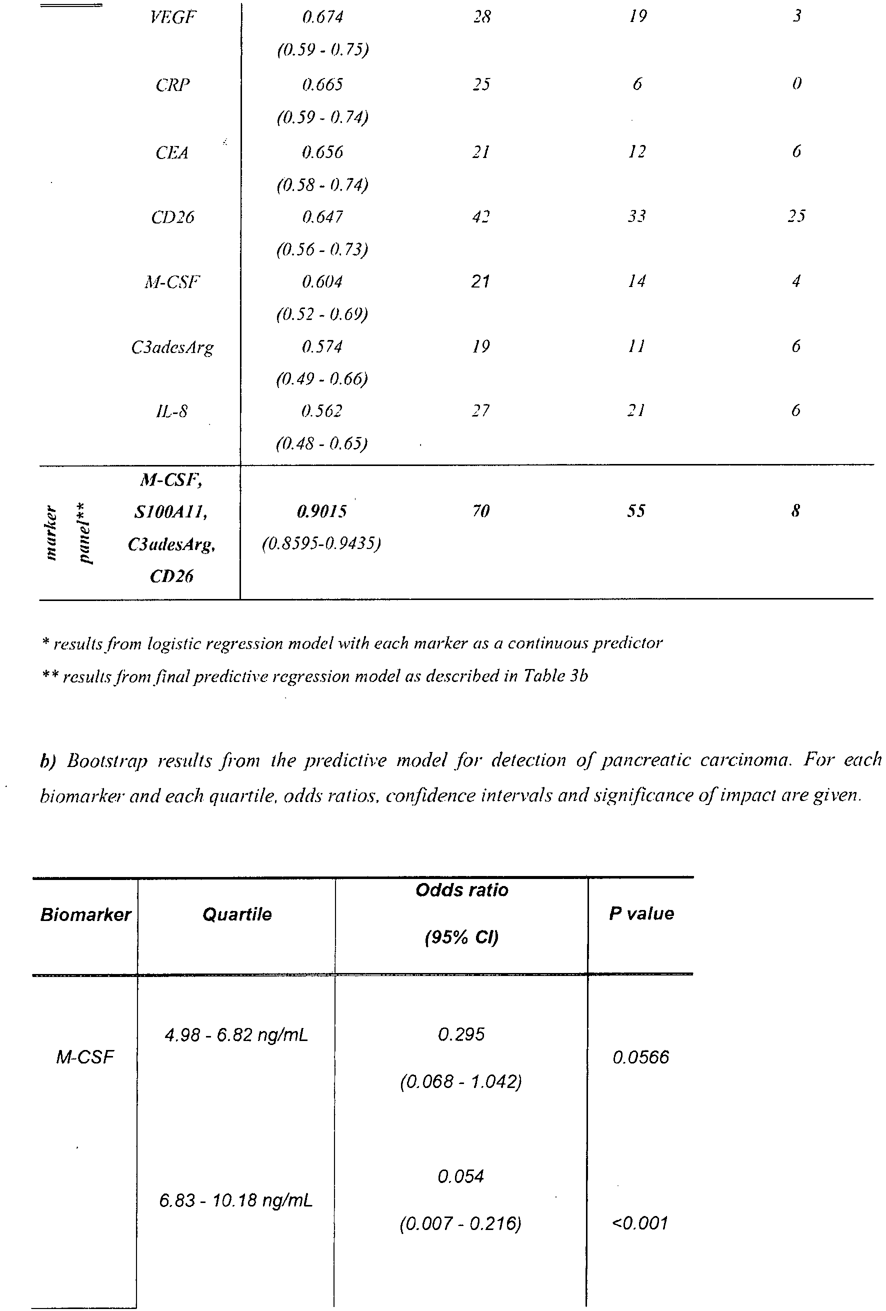

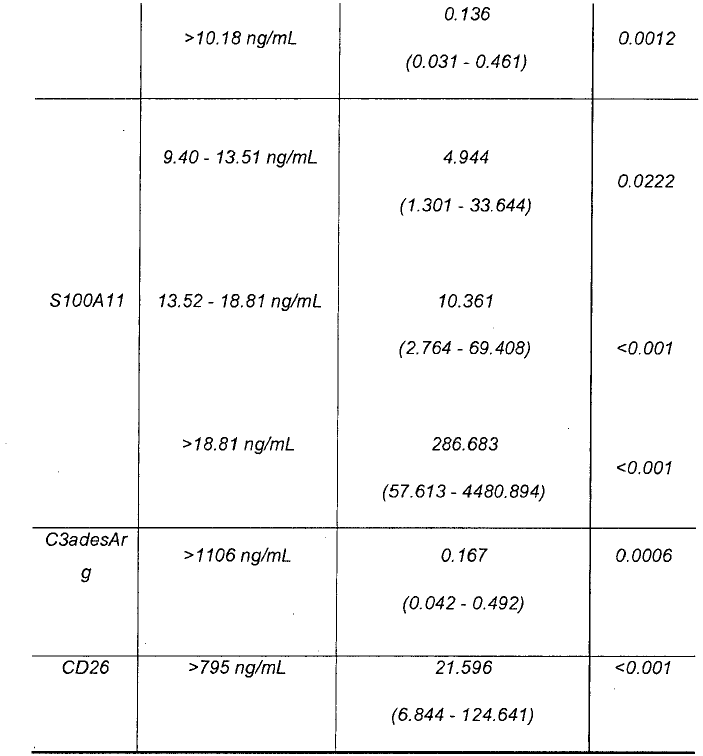

**Table 4 - Summary of clinical data of the study group consisting of pancreatic adenoma patients.**

| *N*/*A* = *not available, IPMN* = *intraductal papillary mucinous neoplasms* | | | | |
|---|---|---|---|---|
| ***Patien*** ***t*#** | ***Grade of dysplasia*** | ***Histology*** | ***Adenoma size (cm)*** | ***Adenoma localisation*** |
| *1* | *N*/*A* | *microcystic cystadenoma* | *11,5* | *head* |
| *2* | *low* | *IPMN* | *5* | *tail* |
| *3* | *N*/*A* | *N*/*A* | *N*/*A* | *N*/*A* |
| *4* | *low* | *papillary tubular adenoma* | *2* | *N*/*A* |
| *5* | *N*/*A* | *pseudocystic adenoma* | *2* | *head* |
| *6* | *N*/*A* | *N*/*A* | *N*/*A* | *tail* |
| *7* | *N*/*A* | *IPMN* | *5* | *head* |
| *8* | *N*/*A* | *mucinous cystadenoma* | *9* | *tail* |
| *9* | *low* | *IPMN* | *6* | *head* |
| *10* | *N*/*A* | *multicystic-mucinous cystadenoma* | *8* | *tail* |
| *11* | *N*/*A* | *endocrine microadenoma* | *0,4* | *tail* |
| *12* | *N*/*A* | *microcystic-serous cystadenoma* | *3* | *head* |
| *13* | *N*/*A* | *cystadenoma* | *1,5* | *N*/*A* |
| *14* | *low* | *IPMN* | *N*/*A* | *head* |
| *15* | *N*/*A* | *pseudocystic adenoma* | *N*/*A* | *N*/*A* |

**Table 5 - Spearman's Correlation coefficient ρ and P-valuefor a) healthy patients and b) pancreatic carcinoma patients.**

| ***a)*** | | | | |
|---|---|---|---|---|
| | ***M-CSF*** | ***S100A11*** | ***C3dArg*** | ***CD26*** |
| ***M-CSF*** | *n.a.* | *ρ* = *0.217* | | *ρ* = *-0.235* |
| | | *P* = *0.029* | | *P* = *0.017* |
| ***S100A11*** | *ρ* = *0.217* | *n.a.* | *ρ* = *0.291* | |
| | *P* = *0.029* | | *P* = *0.003* | |
| ***C3adesArg*** | | *ρ* = *0.291* | *n.a.* | |
| | | *P* = *0.003* | | |
| ***CD26*** | *ρ* = *-0.235* | | | *n*.*a.* |
| | *P* = *0.017* | | | |

| ***b)*** | | | | |
|---|---|---|---|---|
| | ***M-CSF*** | ***S100A11*** | ***C3dArg*** | ***CD26*** |
| ***M-CSF*** | *n.a.* | *ρ* = *0.281* | | |
| | | *P* = *0.01* | | |
| ***S100A11*** | *ρ* = *0.281* | *n.a.* | *ρ* = *0.250* | *ρ* = *-0.292* |
| | *P* = *0.01* | | *P* = *0.022* | *P* = *0.007* |
| ***C3adesArg*** | | *ρ* = *0.250* | *n.a.* | |
| | | *P* = *0.022* | | |
| ***CD26*** | | *ρ* = *-0.292* | | *n*.*a*. |
| | | *P* = *0.007* | | |

## Claims

1. A method of determining whether a patient has, or is at risk of developing pancreatic cancer comprising,
i) determining the protein level of the biomarkers S100A11, M-CSF, C3adesArg and CD26 in an ex vivo blood, plasma or serum sample taken from the patient and,
ii) establishing the statistical significance of the concentration of the biomarkers.

2. The method of claim 1 wherein the levels of one or more additional biomarkers selected from IL-8, CEA, VEGF and CRP are also determined.

3. The method according to Claims 1 or 2 wherein each of the biomarker concentration values is inputted into a statistical methodology to produce an output value that indicates whether the patient has or is at risk of developing pancreatic cancer.

4. The method of claim 3 wherein the statistical methodology used is logistic regression, decision trees, support vector machines, neural networks, random forest or another machine learning algorithm.

5. The method of any preceding claim wherein the patient sample is contacted with a solid-state device comprising a substrate having an activated surface on to which is immobilized, in discrete areas of said activated surface, one or more antibodies specific to S100A11, M-CSF, C3adesArg and CD26.

## Patentansprüche

1. Ein Verfahren zum Bestimmen, ob ein Patient ein Pankreaskarzinom oder ein Pankreaskarzinom aufweist, umfassend
i) Bestimmen des Proteinspiegels der Biomarker S100A11, M-CSF, C3adesArg und CD26 in einer ex vivo entnommenen Blut-, Plasma- oder Serumprobe des Patienten und
ii) Ermittlung der statistischen Signifikanz der Konzentration der Biomarker.

2. Verfahren nach Anspruch 1, wobei die Spiegel von einem oder mehreren zusätzlichen Biomarkern, ausgewählt aus IL-8, CEA, VEGF und CRP ebenfalls bestimmt werden.

3. Verfahren nach Anspruch 1 oder 2, wobei jeder der Biomarker-Konzentrationswerte in eine statistische Methodologie eingegeben wird, um einen Ausgabewert zu erzeugen, der anzeigt, ob der Patient ein Risiko für die Entwicklung von Bauchspeicheldrüsenkrebs hat oder besteht.

4. Verfahren nach Anspruch 3, wobei die verwendete statistische Methodologie eine logistische Regression, Entscheidungsbäume, Unterstützungsvektormaschinen, neuronale Netzwerke, Zufallswald oder ein anderer maschineller Lernalgorithmus ist.

5. Verfahren nach einem der vorhergehenden Ansprüche, wobei die Patientenprobe mit einem Festkörper-Vorrichtung in Kontakt gebracht wird, umfassend ein Substrat mit einer aktivierten Oberfläche, auf, an das immobilisierte, in diskrete Bereiche der Oberfläche aktiviert ist, einen oder mehrere Antikörper spezifisch für S100A11, M-CSF, C3adesArg und CD26.

## Revendications

1. Méthode pour déterminer si un patient a ou est à risque de développer un cancer du pancréas comprenant
i) déterminer le niveau de protéine des biomarqueurs S100A11, M-CSF, C3adesArg et CD26 dans un échantillon de sang, de plasma ou de sérum ex vivo prélevé sur le patient et,
ii) établir la signification statistique de la concentration des biomarqueurs.

2. Procédé selon la revendication 1, dans lequel les niveaux d'un ou plusieurs biomarqueurs additionnels choisis parmi IL-8, CEA, VEGF et CRP sont également déterminés.

3. Procédé selon les revendications 1 ou 2, dans lequel chacune des valeurs de concentration de biomarqueurs est introduite dans une méthodologie statistique pour produire une valeur de sortie qui indique si le patient a ou est à risque de développer un cancer du pancréas.

4. Procédé selon la revendication 3, dans lequel la méthodologie statistique utilisée est la régression logistique, les arbres de décision, les machines à vecteurs de support, les réseaux de neurones, les forêts aléatoires ou un autre algorithme d'apprentissage automatique.

5. Procédé selon l'une quelconque des revendications précédentes dans lequel l'échantillon de patient est mis en contact avec un dispositif à l'état solide comprenant un substrat ayant une surface activée sur laquelle est immobilisée, dans des zones discrètes de ladite surface activée, un ou plusieurs anticorps spécifiques de S100A11, M -CSF, C3adesArg et CD26.
